(19)

Europäisches Patentamt
European Patent Office
Office européen des brevets

(11) **EP 3 650 864 B1**

(12) **EUROPEAN PATENT SPECIFICATION**

(45) Date of publication and mention
of the grant of the patent:
**18.08.2021 Bulletin 2021/33**

(51) Int Cl.:
**G01N 33/84** *(2006.01)*       **G01N 27/414** *(2006.01)*
**G01N 33/487** *(2006.01)*

(21) Application number: **19208442.4**

(22) Date of filing: **12.11.2019**

(54) **METHOD FOR DIAGNOSING CANCER AND KIT THEREFOR**

VERFAHREN ZUR DIAGNOSE VON KREBS UND KIT DAFÜR

PROCÉDÉ DE DIAGNOSTIC DU CANCER ET KIT ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.11.2018 IT 201800010263**

(43) Date of publication of application:
**13.05.2020 Bulletin 2020/20**

(73) Proprietors:
• **Università Degli Studi Magna Graecia Catanzaro**
  **88100 Catanzaro (IT)**
• **Malara, Natalia**
  **88046 Lamezia Terme (CZ) (IT)**
• **Gentile, Francesco**
  **87040 Castrolibero (CS) (IT)**
• **di Fabrizio, Enzo Mario**
  **00199 Roma (RM) (IT)**
• **Coppede, Nicola**
  **43124 Parma (PR) (IT)**

(72) Inventors:
• **MALARA, Natalia**
  **88046 Lamezia Terme (CZ) (IT)**
• **GENTILE, Francesco**
  **87040 Castrolibero (CS) (IT)**
• **COPPEDE, Nicola**
  **43124 Parma (PR) (IT)**

(74) Representative: **Robba, Pierpaolo**
**Interpatent S.R.L.**
**Via Caboto, 35**
**10129 Torino (IT)**

(56) References cited:
• **Natalia Malara: "Early cancer detection using organic electrochemical transistor based on the conductive polymer", Nano Innovation 2016 conference abstracts, 22 September 2016 (2016-09-22), XP055603051, Retrieved from the Internet: URL:http://www.nanoinnovation.eu/2016/prog ramme/22-sep-morning/112-abstract/713-ts-v iii-b-3 [retrieved on 2019-07-08]**
• **FRANCESCO GENTILE ET AL: "Geometrical Patterning of Super-Hydrophobic Biosensing Transistors Enables Space and Time Resolved Analysis of Biological Mixtures", SCIENTIFIC REPORTS, vol. 6, no. 1, 12 January 2016 (2016-01-12), XP055405302, DOI: 10.1038/srep18992**
• **N. MALARA ET AL: "Superhydrophobic lab-on-chip measures secretome protonation state and provides a personalized risk assessment of sporadic tumour", NPJ PRECISION ONCOLOGY, vol. 2, no. 1, 19 November 2018 (2018-11-19), XP055603059, DOI: 10.1038/s41698-018-0069-7**
• **BENOÎT PIRO ET AL: "Fabrication and Use of Organic Electrochemical Transistors for Sensing of Metabolites in Aqueous Media", APPLIED SCIENCES, vol. 8, no. 6, 4 June 2018 (2018-06-04), page 928, XP55695263, CH ISSN: 2076-3417, DOI: 10.3390/app8060928**
• **XENOFON STRAKOSAS ET AL: "The organic electrochemical transistor for biological applications", JOURNAL OF APPLIED POLYMER SCIENCE, vol. 132, no. 15, 7 January 2015 (2015-01-07), pages n/a-n/a, XP055264620, US ISSN: 0021-8995, DOI: 10.1002/app.41735**

EP 3 650 864 B1

## Description

### Technical Field

The present invention relates to a method for diagnosing cancer, more particularly a method the for early diagnosis of cancer.

The invention further relates to a kit for carrying out said method.

### Background Art

Neoplastic disease is still one of the leading causes of death in the world today. 90% of cancer deaths are due to the spread of the disease or the development of metastases.

In this regard, the scientific community has focused economic efforts and scientific attention on a group of cells, defined as circulating tumor cells (CTCs) that are available through a non-invasive procedure, i.e. a simple blood draw, and have proved to have significant clinical utility in the prognostic evaluation of the patient and in the early detection of cancer. To date, the diagnosis and assessment of the risk of becoming ill with cancer are compromised by the use of instruments characterized by moderate sensitivity and inadequate accuracy. Similarly, the most advanced radiological techniques do not achieve the spatial resolution necessary to detect a tumor in the early molecular stages of its development. On the other hand, the great variety of types of cancer and the different forms that cancer takes in individual patients are also the main cause of failure of advanced formulations also in the field of nanomedicine (Heidi Ledford, Bankruptcy of nanomedicine firm worries drug developers, Nature 533:304-305, 2016).

Devices and essays have been developed for the detection of cancers (Kaiser J. Liquid biopsy' for cancer promises early detection. Science. 19;359-259. (2018)) e per l'analisi e il monitoraggio della progressione del cancro (Hong X, Sullivan RJ, Kalinich M, Kwan TT, Giobbie-Hurder A, Pan S, LiCausi JA, Milner JD, Nieman LT, Wittner BS,et al. Molecular signatures of circulating melanoma cells for monitoring early response to immune checkpoint therapy. Proc Natl Acad Sci USA. 10:2467-2472. (2018)) and for the identification of relevant personalized pharmacological targets (Brady, S.W., McQuerry, J.A., Qiao, Y., Piccolo, S.R., Shrestha, G., Jenkins, D.F., Layer, R.M., Pedersen, B.S., Miller, R.H., Esch, A., et al. Combating subclonal evolution of resistant cancer phenotypes. Nat. Commun. 8, 1231. (2017).). However, no current approach is competitive with the clinical diagnosis, which is already late compared to the global history of a tumor responsible for little or no control of the neoplastic disease and to its high mortality and health care costs (Carrera PM, Hagop M. K., Blinder V.S. The financial burden and distress of patients with cancer: Understanding and stepping-up action on the financial toxicity of cancer treatment. CA Cancer J Clin. 68,153-165. (2018).

In the abstract titled "Early cancer detection using organic electrochemical transistor based on the conductive polymer" of a presentation taken by Natalia Malara in the Nano Innovation 2016 conference it is disclosed, in general term, the possibility of diagnosing cancer by analysing the peripheral blood secretome using an organic electrochemical transistor.

The object of the present invention is to overcome the problems and limitations of prior art by providing a method for non-invasive cancer diagnosis capable of early detection and diagnosis of cancer with high sensitivity and precision.

These and other objects are achieved with the method for diagnosing cancer and the kit therefor as claimed in the appended claims.

### Summary of the Invention

The method for diagnosing cancer according to the invention allows, through a blood draw and the use of a measuring device of the electrochemical type, to detect the defective metabolism of cells as an indicator of abnormal cell division, proliferation and invasion. By measuring the conductivity variation induced by the proportion of titratable free protons (protonation state) contained in cellular catabolites secreted by circulating tumor cells, the method allows to identify cancer patients with a high degree of accuracy and predictive potential.

The method for diagnosing cancer according to the present invention is therefore based on the measurement of the state of molecular protonation of a secretome produced by cells isolated from the peripheral blood (blood tissue) of an individual.

In particular, the method involves a step of producing the secretome, a step of measuring the state of molecular protonation of the secretome, and a step of processing of the data obtained from the measurement to obtain an early detection of cancer.

The secretome production step requires that it is obtained from a cell culture from peripheral blood of the individual, in which, in particular, a non-haematological cellular component present in the peripheral blood is allowed to expand in the cell culture.

During the culture process, at predetermined intervals, a portion of the cell culture medium is replaced by a fresh culture medium and the gradually replaced portions are stored. After a predetermined culture period, the entire culture medium is taken from the culture and the supernatant is obtained therefrom in a known manner. Subsequently, the previously replaced and stored portions of the culture medium are added to this supernatant, resulting in a mixture that constitutes the final total conditioned medium, which is then filtered, thus obtaining the secretome to be analysed.

The use, in this method of diagnosis, of the secretome obtained from a cell culture from peripheral blood of the individual is particularly advantageous because peripheral blood represents, in personalized medicine, the human sample easiest to collect without modification of the clinical conditions of the individual.

Moreover, thanks to the fact that the culture process used involves the expansion of the non-haematological cellular component present in the peripheral blood, the secretome obtained from this culture process provides information not only on the metabolic and biochemical state of blood cells but also and above all of non-haematological cells carrying valuable information derived from tissues other than haematic tissue.

The step of measuring the state of molecular protonation of the secretome involves the use of a measuring device of the electrochemical type comprising at least one measurement point, or preferably several measurement points, each comprising a conductive polymer channel between a respective first and second electrode. Said channel acts as a conducting channel of a respective transistor and is connected to a gate electrode of the transistor through the secretome. At least one measurement of channel current I(t) is then performed at each measurement point. If several channel current measurements are performed at one measurement point, these are performed at different potentials Vds applied between the first and second electrode of the measurement point. A modulation m and a time constant $\tau$ are obtained from each measurement of channel current I(t). The modulation m is given by a final intensity Ids of channel current normalized with respect to the initial intensity IdsO, and the time constant $\tau$ is given by the time it takes for the channel current I(t) to reach a predetermined percentage of the final intensity Ids. The above values of modulation m and time constant $\tau$ are indicative of the protonation state of the secretome.

The step of measuring the state of molecular protonation of the secretome also provides to make Marangoni convection flows develop in the secretome, before performing said at least one measurement of channel current I(t), so that, when the above measurement of channel current is performed, different molecular species (mainly proteins and nucleic acids) contained in the secretome are distributed in different positions within the secretome according to their dimensions and diffusion coefficients D. These Marangoni flows develop thanks to the fact that the measuring device on which the secretome is positioned has a superhydrophobic surface, so that the secretome positioned on the measuring device assumes an almost spherical drop-like shape, which wets all the measurement points of the measuring device, connecting each measurement point to the gate electrode.

In addition, the step of measuring the state of molecular protonation of the secretome preferably provides to place the secretome drop symmetrically with respect to the plurality of measurement points of the measuring device.

The step of measuring the state of molecular protonation of the secretome further provides to select, among the several pairs of modulation m and time constant $\tau$ obtained from the measurements of channel current I(t) performed at different measurement points, a pair of values of modulation m and time constant $\tau$, said pair being representative of the analyzed secretome. Such selection is effected, for example, by means of statistical analyses of known type.

The step of processing the data obtained from the measurements described above involves classifying a secretome, based on the value of modulation m and time constant $\tau$ associated with that secretome, distinguishing whether a secretome comes from a healthy individual, an individual having cancer or an individual at risk of getting cancer.

In case there are pairs of values of modulation m and time constant $\tau$ associated to respective secretomes of different individuals, the processing step provides to apply clustering algorithms to these pairs of values of modulation m and time constant $\tau$ and to group, by applying said algorithms, these secretomes, classifying them in secretomes from healthy individuals, individuals having cancer or individuals at risk of getting cancer.

**Brief Description of the Drawings**

These and other features and advantages of this invention will be more evident from the following description of preferred embodiments given by way of non-limiting example with reference to the annexed drawings, in which elements designated by same or similar reference numeral indicate elements that have same or similar functionality and construction, and in which:

- Figure 1a is a representation of cells in a cell culture obtained according to the invention from peripheral blood of an individual not having cancer, with its legend;
- Figure 1b shows graphs of the concentration of molecular products released by the cell culture in Figure 1a;
- Figure 2a is a representation of cells in a cell culture obtained according to the invention from peripheral blood of an individual having cancer (see the legend of Figure 1a);
- Figure 2b shows graphs of the concentration of molecular products released by the cell culture in Figure 2a;
- Figure 3a is a representation of cells in a cell culture obtained according to the invention from peripheral blood of an individual having metastatic cancer (see the legend of Figure 1a);

- Figure 3b shows graphs of the concentration of molecular products released by the cell culture in Figure 3a;
- Figure 4a is a perspective view of the measuring device according to the invention for measuring the protonation state of the secretome;
- Figure 4b is a top view of a pillar matrix of the device in Figure 4a;
- Figure 4c is a partial sectional view of a pillar of the device in Figure 4a;
- Figure 5 is a perspective view of a measuring device according to the invention on which a drop of secretome sample solution to be analyzed is placed;
- Figure 6a is a graph of a succession of current measurements associated with a measurement point of the measuring device according to the invention;
- Figure 6b is a graph showing a current measurement and the modulation parameters m and time constant $\tau$ derived from that current measurement;
- Figure 7 is a graph showing representative pairs of m-$\tau$ values of the respective samples analyzed, obtained by the method according to the invention;
- Figure 8 is a graph showing the grouping of the pairs of m-$\tau$ values in Figure 7 in order to distinguish secretome solution samples from healthy individuals, individuals having cancer or individuals at risk of getting cancer.

**Description of Some Preferred Embodiments of the Invention**

A method for the early diagnosis of cancer in an individual based on the measurement of the state of molecular protonation of a secretome produced by cells isolated from the peripheral blood (haematic tissue) of the individual is described below.

The method provides a step of producing the secretome, a step of measuring the state of molecular protonation of the secretome and a step of processing the data obtained from the measurement to obtain an early diagnosis of cancer.

The production of the secretome from peripheral blood requires the application of a protocol of isolation, culture and brief in vitro expansion of the non-haematological cellular component, present in low concentrations, in the peripheral blood, described in Italian patent application No. 10201724609.

In accordance with this protocol, the cell culture is preferably maintained for fourteen consecutive days, with partial replacement of the culture medium. In particular, every 48 hours, preferably, the cell culture is checked and 10% of the total volume of the culture medium is taken and replaced with fresh culture medium. The volumes taken are stored in special cuvettes and preferably stored at 4 °C. At the end of the fourteen days, the total volume of the culture medium is taken, for example by means of a centrifuge at 1870 rpm operated for about fifteen minutes. After centrifugation, the pellet (i.e. the material remaining on the bottom) is separated from the supernatant (i.e. the material remaining in suspension). The volumes of the culture medium previously taken at 48-hour intervals are added to the supernatant taken after centrifugation, resulting in a mixture that constitutes the final total conditioned medium, which is then filtered, stored and preserved preferably at a temperature of -80°C, thus obtaining the secretome to be analyzed.

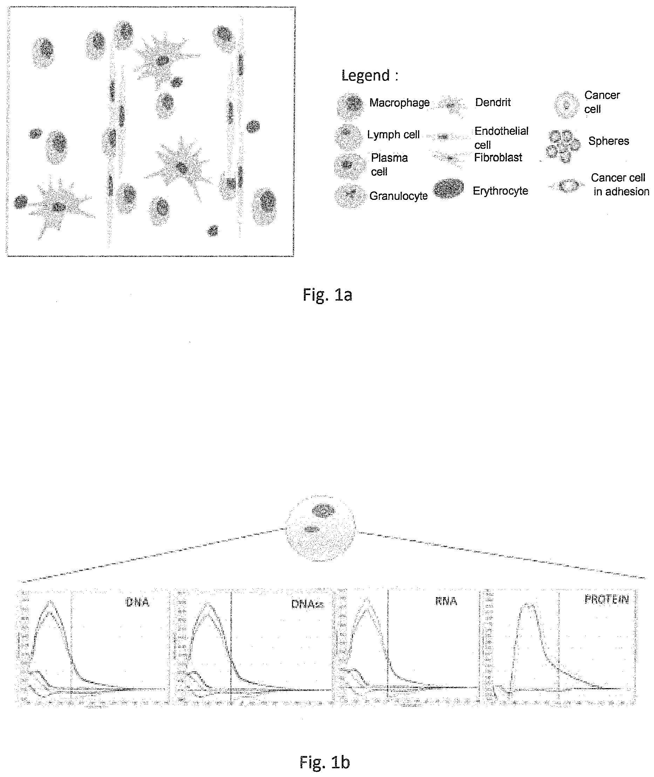

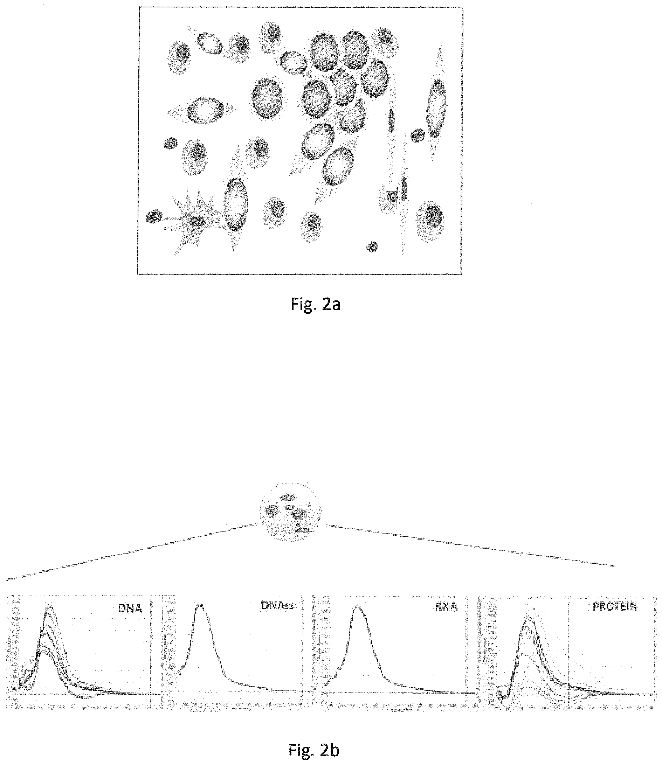

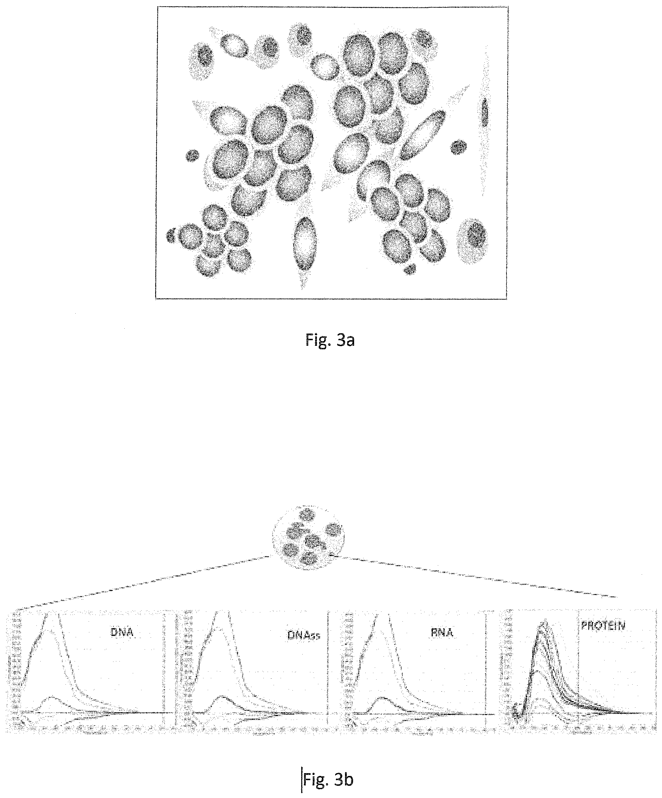

The isolation treatment prior to cell cultivation ensures the presence, in the culture, of a non-haematological component, if present, together with the haematological component proper to the haematic tissue, as shown in Figures 1a, 2a and 3a. This heterogeneous cellular composition determines the secretion of molecules according to the functional, differentiating and metabolic state of the cells that produce them.

The composition of the culture in the extracellular space is characterized by different molecular products (or species) released by the cultured cells. The molecular species present in the secretome are mainly proteins and nucleic acids. With reference to Figures 1b, 2b and 3b, the ratios between the amounts of proteins and nucleic acids vary according to the state of health of the patient from whom the blood sample was taken for culture preparation. Calibration experiments conducted by the Applicant have shown that the protein content of the secretome increases in individuals with cancer and in particular the protein content is related to the degree of cancer (with a Pearson correlation factor r of 0.6). The nucleic acid component includes double-stranded DNA, single-stranded DNA and RNA. The qualitative analysis of the molecular products present in the secretome depends on the type of cells present in the culture and expanding there.

During the cultivation period, the seeded cells divide and increase in number with a duplication time that depends on their replication capacity. By increasing the number of cells over time, the amount of molecules they secrete in the culture medium increases proportionally. If one cell population grows more than another one, the former conditions the culture medium more than the latter and the secretome will have a molecular composition that reflects the cell functions and metabolism of the prevailing cell population.

These considerations underline that, as observed by the Applicant, in a cell culture derived from an individual with cancer, the resulting presence of transformed cells in the culture will tend to characterize the secretome more than the normal cell component because the latter has a lower proliferative capacity than the tumor.

In this regard, the comparison, conducted by the Applicant, between the secretome and the interstitial fluid taken directly from the tumor tissue showed a strong correlation (with a Pearson correlation factor r of 0.9) in terms of types and amounts of proteins present in the two samples taken from the same individual.

The above data support that the composition of the secretome of the cell culture obtained from peripheral blood taken from individuals with neoplastic disease is comparable to that of the interstitial fluid of the primary tumor mass.

Finally, in order to assess whether the characteristics related to ionic conductivity related to the protonation state are different when comparing the secretome obtained from the cell culture derived from the peripheral blood of a healthy individual compared to that of an individual with cancer, the Applicant has studied the isoelectric point of the proteins contained therein, i.e. the pH value at which these proteins have no net electrical charge. This analysis has shown that the isoelectric point of the secretome of cell cultures obtained from the peripheral blood of ill individuals is different from the same derived from healthy individuals.

On the basis of these premises, the secretome obtained according to the above-illustrated procedure is analyzed by measuring the protonation state thereof. This analysis is carried out by means of a measuring device 10 capable of highlighting the different ionic conductivity of the ionized molecules contained in the secretome and of providing, depending on the concentration and intermolecular complexation capacity of these molecules, information suitable for early detection of cancer.

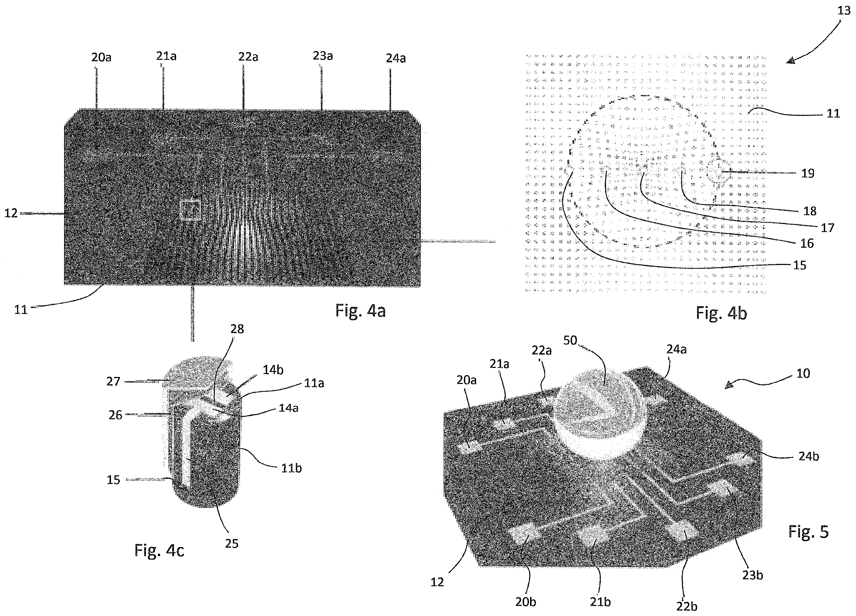

The measuring device 10 for measuring the protonation state of the secretome, shown in Figures 4a-4c and 5, is a chip made up of micrometric pillars 11 made of polymer resist, for example SU8, each having a top surface 11a and a side surface 11b and being arranged according to a hexagonal matrix 13 with variable pitch (Figures 4a and 4b), in which the pitch is smaller in the central portion of the device. The pillars 11 are placed on a silicon substrate 12 where several pairs of independent electrical tracks are integrated, for example five pairs 20a-b, 21a-b, 22a-b, 23a-b, 24a-b, preferably made of gold, connected to a measuring probing station, external to the device 10.

With particular reference to Figure 4c, a pair of nano-electrodes 14a-b is provided on the top surface 11a of some of the pillars 11 of the device. The pillars 11 with nano-electrodes 14a-b are the so-called measurement pillars 15-19 (measurement points) and are equal in number to the pairs of electric tracks 20a-b, 21a-b, 22a-b, 23a-b, 24a-b, i.e. each measurement pillar 15-19 is associated with a respective pair of electric tracks 20a-b, 21a-b, 22a-b, 23a-b, 24a-b. In particular, each electrode of the pair of electrodes 14a-b of a measuring pillar 15-19 is connected only, through connections 25 running along the lateral surface 11b of the pillar, to a respective track of the pair of tracks 20a-b, 21a-b, 22a-b, 23a-b, 24a-b associated with this measuring pillar 15-19. By using these measuring pillars 15-19, as illustrated below, the ion current of the molecular species of a secretome sample solution placed on the device 10 is measured.

The entire measuring device 10 is covered with a thin film of conductive polymer 26, preferably PEDOT:PSS, possibly with solvents suitable for increasing conductivity (e.g. ethylene glycol 10% by weight and DBSA (dodecyl benzene sulfonic acid) 0.1% by weight), which creates a conducting channel 28 between the nano-electrodes 14a-b of a measuring pillar 15-19. The device 10 is finally covered with a thin hydrophobic polymer film 27, for example a film made of $C_4F_8$ (Teflon).

The geometric arrangement of the pillars 11 and the hydrophobic film 27 make the device 10 superhydrophobic, so that a biological solution placed on the device has contact angles close to 180°, assuming an almost spherical drop-like shape 50.

Thanks to the superhydrophobic properties of the device 10, within the drop 50 of secretome sample solution, depending on its curvature, convective flows, so-called Marangoni flows, are developed that transport the molecular species contained within the drop in a differential way, modifying the distribution thereof. In particular, the molecular species contained within the drop move according to their diffusion coefficient D and their dimensions, as described by Einstein's relation $D=K_BT/6\pi\eta a$ (where $K_B$ is Boltzmann's constant, T is the temperature, $\eta$ is the viscosity of the solution and a is the radius of the molecule in solution), this movement distributing the molecular species over the surface of the measuring device 10 at different points.

In addition, the variable pitch of the grating of pillars 11, with higher density of pillars in the central part of the device 10, allows self-centering of the drop 50 with respect to the substrate (Figure 5). This is because, in a variable pitch grating, the surface energy density, and with it the hydrophobicity of the substrate 12, varies according to the pitch. In particular, in the center of the device 10, the pitch and hydrophobicity are minimal, so the drop 50, based on the principle of minimizing the free energy of systems, will be positioned in the center of the device 10, centering itself. Thanks to the self-centering of the drop 50 with respect to the device 10, the measuring pillars 15-19 are positioned symmetrically with respect to the drop itself. In addition, the measuring pillars 15-19 are positioned at such a distance that the drop 50 wets them all.

The distributed arrangement of the measurement points 15-19 allows measurements of the ion current of the secretome sample solution to be made with a spatial as well as a temporal resolution. To carry out these ion current measurements, a multiple organic electrochemical transistor geometry with a common gate has been applied, in which the channel 28 made of PEDOT:PSS between the nano-electrodes 14a-b of each measuring pillar 15-19 is the conducting channel of each transistor, and all the conducting channels 28 are put in contact, through the drop 50 of secretome sample solution, with a common gate, for example a conductive filament made of platinum (not shown), placed on top of the drop 50.

The device 10 therefore exploits the combination of its superhydrophobic properties, combined with the characteristics of the drop 50 of secretome sample solution, and a matrix of organic electrochemical sensors 15-19 based on a transistor architecture.

Fixed potentials are sequentially applied to the pairs of electric tracks 20a-b, 21a-b, 22a-b, 23a-b, 24a-b of each transistor or measurement point 15-19, so that a current (Ids) is passed in the channel 28 of each transistor. This current is measured separately for each of the channels 28, i.e. for each of the measurement points 15-19. By applying a positive potential to the gate electrode (Vgs=1V) the ions in the drop 50 of secretome sample solution are forced to enter the channel 28 made of PEDOT:PSS of the transistors. Since it is known that the conductivity of PEDOT:PSS decreases in the presence of positive ions, a decrease in current proportional to the concentration of ions in the vicinity of the channels 28 is obtained in the channels 28. In particular, the application of the positive potential (Vgs) to the gate electrode, determines that the positive ions (M+) of the drop 50 penetrate into the channel 28 made of PEDOT:PSS using its de-doping as per the following reaction:

$$PEDOT+:PSS- + M+ + e- => PEDOT0 + M+: PSS-$$

The de-doping process causes a decrease in the value of the module of the channel current |Ids| due to a decrease in the holes available for conduction. The positive ion M+ allows the reduction of the oxidized state of the PEDOT+ to PEDOT0. This process is reversible and when the Vgs potential is switched off the ions return to diffuse in the drop 50 and bring the polymer back to the previous doping state.

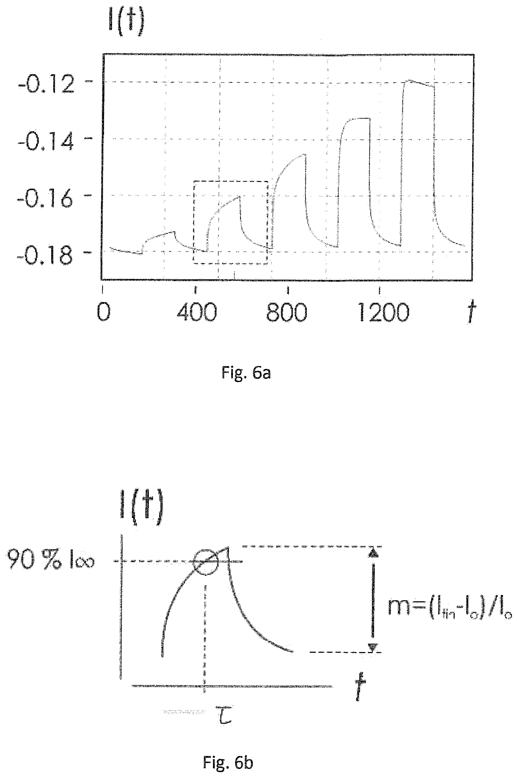

The penetration, or injection, of the ions into the conducting channel 28 occurs when the gate potential Vgs is switched on, generating a signal of reduction of the channel current I(t) with exponential trend (Figure 6b), whose relative variation or modulation m (m=Ids-Ids0/Ids0) depends on the concentration of ions and whose time constant $\tau$ depends on the charge and mass (and therefore on the diffusion coefficient D) of the ions. The modulation m is the final intensity of current Ids normalized with respect to the initial IdsO, and is therefore a dimensionless number. The time constant $\tau$ indicates the time it takes for the system to reach, for example, 90% of the final response, and is measured in seconds.

With reference to Figure 6a, for each transistor (or, similarly, for each measurement point 15-19), different sequences of gate potentials are applied, thus obtaining a signal of reduction of the channel current I(t), for each measurement point 15-19, given by a sequence of exponentials (Figure 6a). For each measurement point 15-19 a succession of values of modulation m and time constant $\tau$ is obtained, which depend on the characteristics of the ions near the measurement point 15-19 and which define, in a plane m-$\tau$, trajectories associatable, through a qualitative evaluation, with a particular molecular species present near the measurement point 15-19. Alternatively, it is also possible to make, for each measurement point 15-19, an I(t) measurement corresponding to a single gate potential Vgs, thus obtaining a signal I(t) comprising a single exponential and therefore a single m-$\tau$ pair.

By measuring the values of modulation m and time constant $\tau$, information on the molecular species in solution is obtained. In particular, m is inversely proportional to mass and directly proportional to the charge of a molecular ion whereas $\tau$ is directly proportional to mass and inversely proportional to the charge of a molecular ion.

By crossing the above qualitative data (association of a trajectory in the m-$\tau$ plane with a particular molecular species) and quantitative data (values of m and $\tau$) obtained for each measurement point 15-19 of the measuring device 10, a detection is obtained that allows comparison of the ionic strength at the different measurement points 15-19 of the measuring device 10.

The method for measuring the protonation state (PS) of a secretome sample solution and the subsequent processing for obtaining a diagnosis is described in more detail below.

A change in current I(t) over time measured by the measuring device 10 is associated with the transport of ionized molecular species within the solution and depends in particular on five parameters (p1-p5), namely the diffusion coefficient D (p1), the charge z of the molecular species within the solution (p2), the Marangoni convection flows carrying the species within the solution drop 50 (p3), the position and density of the measurement points 15-19 on the substrate 12 of the device 10 (p4) and the intensity of the electromotive force (EMF), i.e. the potential Vds applied between the first 14a and the second 14b electrode of the measurement points 15-19 for the measurement of the current I(t)(p5).

The parameters p1 and p2 are physical characteristics of the molecular species under examination, which are to be determined and which encode the protonation state of specific molecular species of the secretome. The parameters p3, p4, p5 are manufacturing parameters and can be suitably modulated by choosing the size of the drop 50 and the hydrophobicity of the surface 27 of the device 10, the number and type of measurement points 15-19 in the device 10, the intensity of the electromotive force. By modulating the parameters p3, p4, p5 it is possible to optimize the response of the device 10 and maximize the sensitivity of the function I(t).

The steps of the protonation state (PS) measurement method are as follows:

(A) After a careful analysis, the parameters p3, p4, p5 are chosen that guarantee the best response of the device 10.

(B) Measurements are made on different sample solutions in a small range of parameters around p3, p4, p5 and each measurement is coded in a current-time curve I(t), as described above.

(C) The curves I(t) are analyzed and the two variables modulation m and time constant $\tau$ are extracted from each curve, which are characteristic of a specific state of protonation.

(D) Using a known statistical analysis (ANOVA) for each sample solution, a single pair of m-$\tau$ values (representative pair) out of all the measured pairs is obtained.

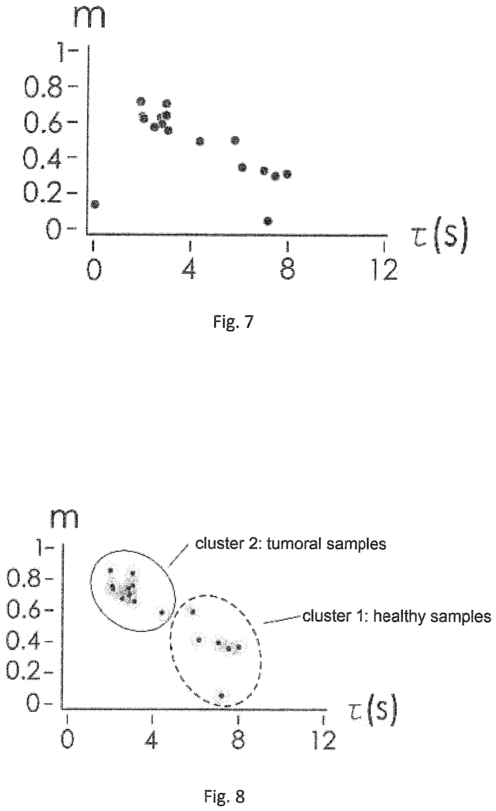

(E) The representative pairs of each sample solution analyzed are reported in an m-$\tau$ plane. Different sample solutions, which express different protonation states (Ps), will be characterized by a different representative m-$\tau$ pair and will therefore be positioned in different areas of the m-$\tau$ plane (Figure 7).

(F) Clustering algorithms based on Euclidean metrics are applied to the representative pairs shown in the m-$\tau$ diagram. These algorithms group the sample solutions into distinct groups on the basis of their similarity. The sample solutions are thus classified into solutions from healthy individuals, individuals having cancer or individuals at risk of getting cancer on the basis of the group to which they belong (Figure 8).

**Claims**

1. A method for diagnosing cancer in an individual, comprising the steps of:

    - obtaining a secretome from a cell culture obtained from peripheral blood of the individual, wherein a non-hematological cell component present in peripheral blood has been allowed to expand in said cell culture;
    - performing at least one measurement of channel current I(t) at at least one measurement point (15-19) comprising a channel of conductive polymer (28) located between a respective first (14a) and second (14b) electrode, said channel (28) being arranged to act as a conducting channel of a transistor and being connected to a gate electrode of said transistor through the secretome;
    - obtaining, from said at least one measured channel current I(t), a modulation m, given by a final intensity Ids of the channel current normalized with respect to the initial intensity IdsO, and a time constant $\tau$ given by the time it takes for the channel current I(t) to reach a predetermined percentage of the final intensity Ids, said values of m and $\tau$ being indicative of the protonation state of the secretome;
    - on the basis of the obtained values of modulation m and time constant $\tau$, classifying the secretome as a secretome from a healthy individual, from an individual having cancer, or from an individual at risk of getting cancer.

2. The method according to claim 1, wherein said step of obtaining a secretome from a cell culture obtained from peripheral blood of the individual comprises the step of:

    - replacing, at predetermined intervals, a portion of culture medium of the cell culture with a fresh culture medium, thus obtaining a plurality of replaced portions of culture medium;
    - after a predetermined culture period, taking the whole culture medium of the culture and obtaining the supernatant therefrom;
    - adding the replaced portions of culture medium to said supernatant.

3. The method according to claim 1 or 2, further comprising the step of:

    - making Marangoni convection flows develop within the secretome before said step of performing at least one measurement of channel current I(t), so that, when said at least one measurement of channel current I(t) is performed, different molecular species contained within the secretome are distributed in different positions within the secretome depending on their dimensions and diffusion coefficients D.

4. The method according to claim 3, wherein said step of making Marangoni convection flows develop within the secretome provides for placing the secretome onto a superhyrophobic surface (27).

5. The method according to any of the preceding claims, wherein said at least one measurement of channel current I(t) is performed at a plurality of measurement points (15-19).

6. The method according to claim 5, further comprising the step of wetting the plurality of measurement points (15-19) with one and the same secretome drop (50), said one and the same drop (50) connecting each measurement point (15-19) to the gate electrode.

7. The method according to claim 6, further comprising the step of placing the secretome drop (50) symmetrically with respect to said plurality of measurement points (15-19).

8. The method according to any of the preceding claims, wherein at a measurement point (15-19) a plurality of measurements of channel current I(t) are performed at different potentials Vds applied between said first (14a) and second (14b) electrode between which the channel of conductive polymer (28) is located.

9. The method according to claim 5 or 8, further comprising the step of selecting, among the several pairs of modulation m and time constant $\tau$ obtained from the measurements of channel current I(t) performed at different measurement points, a pair of values of modulation m and time constant $\tau$, said pair being representative of the secretome.

10. The method according to any of the preceding claims, wherein said step of classifying the secretome as a secretome from a healthy individual, from an individual having cancer, or from an individual at risk of getting cancer provides for:

   - applying clustering algorithms to values of modulation m and time constant $\tau$ retrieved from secretomes obtained from different individuals, and
   - grouping, by applying said clustering algorithms, said secretomes obtained from different individuals, classifying said secretomes as secretomes from healthy individuals, from individuals having cancer or from individuals at risk of getting cancer.

11. Kit for the early diagnosis of cancer in an individual, comprising:

   - means for obtaining a secretome from a cell culture obtained from peripheral blood of an individual by allowing a non-hematological cell component present in the peripheral blood to expand in the cell culture;
   - a measuring device (10) comprising at least one measurement point (15-19), said measurement point (15-19) comprising a channel of conductive polymer (28) located between a respective first (14a) and second (14b) electrode, said channel (28) being arranged to act as a conducting channel of a transistor and being connectable to a gate electrode of said transistor through the secretome;
   - means for performing clustering algorithms, wherein said means are adapted for processing data obtained by the measuring device (10) and performing the classification specified in claim 1.

**Patentansprüche**

1. Verfahren zur Diagnose von Krebs in einem Individuum mit den Schritten:

   - Gewinnen eines Sekretoms aus einer Zellkultur, die aus peripherem Blut des Individuums erhalten wurde, wobei man eine nicht hämatologische Zellkomponente, die in dem peripherem Blut vorhanden ist, sich in der Zellkultur hat ausbreiten lassen,
   - Durchführen von wenigstens einer Messung eines Kanalstroms I(t) an wenigstens einem Messpunkt (15-19), der einen Kanal leitfähigen Polymers (28) aufweist, der zwischen einer ersten (14a) und einer zweiten (14b) Elektrode angeordnet ist, wobei der Kanal (28) so angeordnet ist, dass er als leitender Kanal eines Transistors wirkt und über das Sekretom an die Gate Elektrode des Transistors angeschlossen ist,
   - Gewinnen aus dem wenigstens einen gemessenen Kanalstrom I(t) einer Modulation m, die durch eine endgültige Intensität Ids des Kanalstroms gegeben ist, der in Bezug auf die anfängliche Intensität Ids0 normalisiert ist, und einer Zeitkonstanten $\tau$, die durch die Zeit gegeben ist, die es dauert, bis der Kanalstrom I(t) einen vorgegebenen Prozentsatz der endgültigen Intensität Ids erreicht, wobei die Werte von m und $\tau$ den Protonierungszustand des Sektretoms angeben,
   - auf der Basis der erhaltenen Werte der Modulation m und der Zeitkonstante $\tau$ Klassifizieren des Sekretoms als ein Sekretom eines gesunden Individuums, eines Individuums, das Krebs hat oder eines Individuums, das gefährdet ist, Krebs zu bekommen.

2. Verfahren nach Anspruch 1, wobei der Schritt des Gewinnens eines Sekretoms aus einer Zellkultur, die aus peripherem Blut des Individuums erhalten wurde, den Schritt aufweist:

   - Ersetzen eines Teils eines Zellkulturmediums der Zellkultur mit frischem Zellkulturmedium in vorgegebenen Intervallen, wodurch mehrere ersetzte Teile von Zellkulturmedium gewonnen werden,
   - nach einer vorgegebenen Kultivierungsdauer Entnehmen des gesamten Kulturmediums der Kultur und Gewinnen eines Überstands davon,
- Hinzufügen der ersetzten Teile des Kulturmediums zu dem Überstand.

3. Verfahren nach Anspruch 1 oder 2 zusätzlich mit dem Schritt:

- Erzeugen von Marangoni-Konvektionsströmungen in dem Sekretom, bevor der Schritt des Durchführens der wenigstens einer Messung des Kanalstroms I(t), so dass bei Durchführung der wenigstens einen Messung des Kanalstroms I(t) verschiedene in dem Sekretom enthaltene Moleküle in Abhängigkeit von ihren Abmessungen und Diffusionskoeffizienten D an verschiedenen Stellen in dem Sekretom verteilt werden.

4. Verfahren nach einem der Anspruch 3, wobei der Schritt des Erzeugens von Marangoni-Konvektionsströmungen in dem Sekretom vorsieht, dass das Sekretom auf einer superhydrophoben Oberfläche (27) platziert wird.

5. Verfahren nach einem der vorstehenden Ansprüche, wobei die wenigstens eine Messung des Kanalstroms I(t) an mehreren Messpunkten (15-19) durchgeführt wird.

6. Verfahren nach Anspruch 5 zusätzlich mit Schritt des Benetzens der Messpunkte (15-19) mit ein und demselben Sekretomtropfen (50), wobei der ein und derselbe Tropfen (15) jeden Messpunkt (15-19) mit der Gate-Elektrode verbindet.

7. Verfahren nach Anspruch 6, zusätzlich mit dem Schritt des Anordnens des Sekretomtropfens (15) symmetrisch in Bezug auf die Messpunkte (15-19).

8. Verfahren nach einem der vorstehenden Ansprüche, wobei an wenigstens einem Messpunkt (15-19) mehrere Messungen des Kanalstroms I(t) bei verschiedenen Potentialen Vds durchgeführt werden, die zwischen die erste Elektrode (14a) und die zweite Elektrode (14b) angelegt werden, zwischen denen der Kanal leitfähigen Polymers (28) angeordnet ist.

9. Verfahren nach Anspruch 5 oder 8, zusätzlich mit dem Schritt des Auswählens aus verschiedenen Paaren von Modulationen m und Zeitkonstante $\tau$, die aus den Messungen des Kanalstroms I(t) gewonnen wurden, die an verschiedenen Messpunkten durchgeführt wurden, eines Wertepaares von Modulation m und Zeitkonstante $\tau$, das für das Sekretom repräsentativ ist.

10. Verfahren nach einem der vorstehenden Ansprüche, wobei der Schritt des Klassifizierens des Sekretoms als ein Sektretom von einem gesunden Individuum, von einem Individuum, das Krebs hat, oder von einem Individuum, das gefährdet ist Krebs zu bekommen, vorsieht:

- Anwenden von Clustering-Algorithmen auf Werte von Modulation m und Zeitkonstante $\tau$, die von Sekretomen gewonnen wurden, die von verschiedenen Individuen stammen, und
- Gruppieren der Sekretome, die von verschiedenen Individuen gewonnen wurden durch Anwenden der Clustering-Algorithmen, Klassifizieren der Sektretome als Sektretome von gesunden Individuen, von Individuen, die Krebs haben, und von Individuen, die gefährdet sind, Krebs zu bekommen.

11. Kit für die Frühdiagnose von Krebs in einem Individuum mit:

- Mitteln zum Gewinnen eines Sekretoms aus einer Zellkultur, die aus peripherem Blut eines Individuums gewonnen wurde, indem man eine nicht hämatologische Zellkomponente, die in dem peripheren Blut vorhanden ist, sich in der Zellkultur ausbreiten lässt,
- einer Messvorrichtung (10), die wenigstens einen Messpunkt (15-19) aufweist, wobei der Messpunkt (15-19) einen Kanal leitfähigen Polymers (28) aufweist, der zwischen einer entsprechenden ersten (14a) und zweiten (14b) Elektrode angeordnet ist, wobei der Kanal (28) so angeordnet ist, dass er als leitender Kanal einer Transistors wirkt und durch das Sekretom an eine Gate-Elektrode des Transistors angeschlossen werden kann,
- Mittel zum Durchführen von Clustoring-Algorithmen, wobei die Mittel dafür ausgelegt sind, Daten zu verarbeiten, die von der Messvorrichtung (10) erhalten wurden, und die in Anspruch 1 spezifizierte Klassifikation durchzuführen.

**Revendications**

1. Procédé de diagnostic de cancer chez un individu, comprenant les étapes consistant à :

   - obtenir un sécrétome à partir d'une culture cellulaire obtenue à partir de sang périphérique de l'individu, dans lequel on a laissé un composant cellulaire non hématologique présent dans le sang périphérique croître dans ladite culture cellulaire ;
   - réaliser au moins une mesure de courant de canal I(t) au niveau d'au moins un point de mesure (15-19) comprenant un canal de polymère conducteur (28) situé entre une première (14a) et seconde (14b) électrode respective, ledit canal (28) étant agencé pour servir de canal conducteur d'un transistor et étant connecté à une électrode grille dudit transistor par le biais du sécrétome ;
   - obtenir, à partir dudit au moins un courant de canal I(t) mesuré, une modulation m, donnée par une intensité finale Ids du courant de canal normalisé par rapport à l'intensité initiale IdsO, et une constante de temps $\tau$ donnée par le temps nécessaire au courant de canal I(t) pour atteindre un pourcentage prédéterminé de l'intensité finale Ids, lesdites valeurs de m et de $\tau$ étant indicatives de l'état de protonation du sécrétome ;
   - sur la base des valeurs obtenues de modulation m et de constante de temps $\tau$, classifier le sécrétome en tant que sécrétome provenant d'un individu sain, d'un individu souffrant d'un cancer, ou d'un individu présentant un risque d'avoir un cancer.

2. Procédé selon la revendication 1, dans lequel ladite étape consistant à obtenir un sécrétome à partir d'une culture cellulaire obtenue à partir de sang périphérique de l'individu comprend les étapes consistant à :

   - remplacer, à des intervalles prédéterminés, une portion de milieu de culture de la culture cellulaire par un milieu de culture frais, obtenant ainsi une pluralité de portions de milieu de culture remplacées ;
   - après une période de culture prédéterminée, prélever le milieu de culture entier de la culture et obtenir le surnagent à partir de celui-ci ;
   - ajouter les portions de milieu de culture remplacées audit surnageant.

3. Procédé selon la revendication 1 ou 2, comprenant en outre l'étape consistant à :

   - amener des flux de convection Marangoni à se développer au sein du sécrétome avant ladite étape consistant à réaliser au moins une mesure de courant de canal I(t), de sorte que, lorsque ladite au moins une mesure de courant de canal I(t) est réalisée, des espèces moléculaires différentes contenues au sein du sécrétome soient réparties dans des positions différentes au sein du sécrétome en fonction de leurs dimensions et de leurs coefficients de diffusion D.

4. Procédé selon la revendication 3, dans lequel ladite étape consistant à amener des flux de convection Marangoni à se développer au sein du sécrétome prévoit le placement du sécrétome sur une surface superhydrophobe (27).

5. Procédé selon l'une quelconque des revendications précédentes, dans lequel ladite au moins une mesure de courant de canal I(t) est réalisée au niveau d'une pluralité de points de mesure (15-19).

6. Procédé selon la revendication 5, comprenant en outre l'étape consistant à mouiller la pluralité de points de mesure (15-19) avec une seule et même goutte de sécrétome (50), ladite une seule et même goutte (50) connectant chaque point de mesure (15-19) à l'électrode grille.

7. Procédé selon la revendication 6, comprenant en outre l'étape consistant à placer la goutte de sécrétome (50) symétriquement par rapport à ladite pluralité de points de mesure (15-19).

8. Procédé selon l'une quelconque des revendications précédentes, dans lequel au niveau d'un point de mesure (15-19) une pluralité de mesures de courant de canal I(t) sont réalisées à des potentiels Vds différents appliqués entre lesdites première (14a) et seconde (14b) électrodes entre lesquelles est situé le canal de polymère conducteur (28).

9. Procédé selon la revendication 5 ou 8, comprenant en outre l'étape consistant à sélectionner, parmi la pluralité de paires de modulation m et de constante de temps $\tau$ obtenues à partir des mesures de courant de canal I(t) réalisées au niveau de points de mesure différents, une paire de valeurs de modulation m et de constante de temps $\tau$, ladite paire étant représentative du sécrétome.

10. Procédé selon l'une quelconque des revendications précédentes, dans lequel ladite étape consistant à classifier le sécrétome en tant que sécrétome provenant d'un individu sain, d'un individu souffrant d'un cancer, ou d'un individu présentant un risque d'avoir un cancer prévoit :

   - l'application d'algorithmes d'agglomération à des valeurs de modulation m et de constante de temps $\tau$ récupérées à partir de sécrétomes obtenus auprès d'individus différents, et
   - le groupement, en appliquant lesdits algorithmes d'agglomération, desdits sécrétomes obtenus auprès d'individus différents, la classification desdits sécrétomes en tant que sécrétomes provenant d'individus sains, d'individus souffrant d'un cancer ou d'individus présentant un risque d'avoir un cancer.

11. Kit de diagnostic précoce de cancer chez un individu, comprenant :

   - des moyens d'obtention d'un sécrétome à partir d'une culture cellulaire obtenue à partir de sang périphérique d'un individu en laissant un composant cellulaire non hématologique présent dans le sang périphérique croître dans la culture cellulaire ;
   - un dispositif de mesure (10) comprenant au moins un point de mesure (15-19), ledit point de mesure (15-19) comprenant un canal de polymère conducteur (28) situé entre une première (14a) et seconde (14b) électrode respective, ledit canal (28) étant agencé pour servir de canal conducteur d'un transistor et pouvant être connecté à une électrode grille dudit transistor par le biais du sécrétome ;
   - des moyens de réalisation d'algorithmes d'agglomération, dans lequel lesdits moyens sont adaptés pour traiter des données obtenues par le dispositif de mesure (10) et réaliser la classification spécifiée à la revendication 1.

Fig. 1a

Fig. 1b

Fig. 2a

Fig. 2b

Fig. 3a

Fig. 3b

Fig. 4a

Fig. 4b

Fig. 4c

Fig. 5

Fig. 6a

Fig. 6b

Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 10201724609

**Non-patent literature cited in the description**

- **HEIDI LEDFORD.** Bankruptcy of nanomedicine firm worries drug developers. *Nature,* 2016, vol. 533, 304-305
- **KAISER J.** Liquid biopsy' for cancer promises early detection. *Science,* 2018, vol. 19, 359-259
- **HONG X ; SULLIVAN RJ ; KALINICH M ; KWAN TT ; GIOBBIE-HURDER A ; PAN S ; LICAUSI JA ; MILNER JD ; NIEMAN LT ; WITTNER BS.** Molecular signatures of circulating melanoma cells for monitoring early response to immune checkpoint therapy. *Proc Natl Acad Sci USA.,* 2018, vol. 10, 2467-2472
- **BRADY, S.W. ; MCQUERRY, J.A. ; QIAO, Y. ; PICCOLO, S.R. ; SHRESTHA, G. ; JENKINS, D.F. ; LAYER, R.M. ; PEDERSEN, B.S. ; MILLER, R.H. ; ESCH, A. et al.** Combating subclonal evolution of resistant cancer phenotypes. *Nat. Commun.,* 2017, vol. 8, 1231
- **CARRERA PM ; HAGOP M. K. ; BLINDER V.S.** The financial burden and distress of patients with cancer: Understanding and stepping-up action on the financial toxicity of cancer treatment. *CA Cancer J Clin.,* 2018, vol. 68, 153-165